# EUROPEAN PATENT APPLICATION

(11) **EP 2 000 450 A1**
(43) Date of publication of application: **10.12.2008**
(21) Application number: 07290697.7
(22) Date of filing: 05.06.2007
(51) Int. Cl.: C07C 5/333, C07C 15/44, C07C 15/46

(54) **Regeneration and stabilization of dehydrogenation catalyst**

(71) Applicant: Total Petrochemicals France, 92800 Puteaux (FR)
(72) Inventor: Leroux, Patrice, 76310 Sainte Adresse (FR)
(74) Representative: Roufosse, Micheline C.

(57) **Abstract**

The present invention relates to a process for catalytically converting an alkylaromatic hydrocarbon into a vinylaromatic hydrocarbon by directing said alkylaromatic hydrocarbon and steam into a reactor containing dehydrogenation catalyst, said process comprising the steps of:
(a) forming a mixed reactant stream consisting essentially of said alkylaromatic hydrocarbon and steam;
(b) bringing said mixed reactant stream into contact with a dehydrogenation catalyst consisting essentially of iron oxide catalyst promoted with alkali metal and at conditions effective to convert at least a portion of the alkylaromatic hydrocarbon to vinylaromatic hydrocarbon;

wherein an effective amount of an alkali metal compound in the form of a powder is injected continuously or intermittently in at least one of the feedstocks sent to the dehydrogenation catalyst; said effective amount of alkali metal compound being sufficient to maintain substantially constant levels of conversion of alkylaromatic hydrocarbon and selectivity of vinylaromatic hydrocarbon.

## Description

### [Field of the invention]

The present invention relates generally to a method for greatly extending the useful life of a catalyst bed used in the catalytic dehydrogenation of alkylaromatic hydrocarbons while maintaining a very high level of conversion and a very high level of selectivity and without the need to interrupt the conversion process.

### [Background of the invention]

Vinyl benzenes play a particularly important role in the preparation of synthetic plastics and resins. The polymerization of styrenes, for example, to produce polystyrene resins is well known. Styrene and styrene derivatives are typically produced from ethylbenzene materials by dehydrogenation over solid catalysts in the presence of steam.

DE 863 342 describes catalysts made of iron oxide, chromium oxide and potassium salt for dehydrogenation of alkylaromatic hydrocarbons. Dehydrogenation of ethylbenzene to styrene is described. The catalyst can be refreshed by injection of potassium carbonate in the steam in the course of the dehydrogenation but nothing is disclosed about the way of injection : liquid, solid or powder.

US 4590324 relates to a method of dehydrogenating an alkylaromatic compound containing at least two carbon atoms and at least one alkyl group to an alkenylaromatic which comprises contacting the alkylaromatic with a catalyst comprising copper on a support comprising aluminum borate. Either prior to or after the conversion of the copper aluminum borate to the catalyst comprising finely divided copper on a substrate or support comprising aluminum borate, the aluminum borate can be treated or doped with an alkali metal or alkaline earth metal compound for use in the dehydrogenation. Doping is particularly advantageous for conversion of ethylbenzene to styrene. The alkali metal or alkaline earth metal compound can be dry blended with the copper aluminum borate or copper on aluminum borate; dissolved in a suitable solvent, preferably water, mixed with the copper aluminum borate or copper on aluminum borate and dried; or aqueous solutions of same can be added to feedstocks going to a reactor containing the copper aluminum borate or copper on aluminum borate.

DD 298 353 describes a method to regenerate dehydrogenation catalysts made of iron oxide and potassium oxide. These catalysts are used to dehydrogenation of ethylbenzene to styrene. In said method the dehydrogenation is shut down; a solution of potassium ion is sent with steam on the catalyst at a temperature between the dehydrogenation temperature and 30°C above; after said potassium addition the temperature of the catalyst is rised by 30 to 40°C above the catalyst temperature during one or two hours; then dehydrogenation is resumed.

US 5695724 describes a dehydrogenation catalyst regeneration and/or stabilization method comprising the steps of continually, that is either continuously or intermittently, adding to a reactant feedstream an effective mount of an alkali metal compound during continuation of the dehydrogenation process. The method of this prior art may also include the step of gradually increasing the reaction zone temperature. The method of this prior art can be utilized, for example in the catalytic dehydrogenation of ethylbenzene to styrene, to achieve substantially steady-state reaction conditions at high levels of conversion and selectivity. The alkali metal compound can be injected in the form of an aqueous solution (col 7 lines 59-60). At col 8 second § it is explained that the addition method is to add the alkali metal compound in dry, solid, powdered form to a reactant stream. Nevertheless only a vaporization method and injection of an aqueous solution are described. In the vaporization method a solid lump of or a vessel containing the alkali metal compound in solid, liquid or solution form, can be placed in the path of the heated reactant stream and allowed to gradually vaporize into the passing stream. Still another cited method is to inject the alkali metal compound in a liquid form into the reactant stream. Still another cited method is to vaporize the alkali metal compound and inject the vapor into the reactant stream. In the examples solid KOH has been put in the reactor or in the superheater and KOH vaporized continuously.

US6936743 relates to an improvement in a process to prepare a vinyl aromatic hydrocarbon prepared by dehydrogenating an alkyl aromatic hydrocarbon wherein the dehydrogenation is performed using a dehydrogenation catalyst. The invention is particularly useful with processes wherein the catalyst is one containing iron and at least one alkali metal compound, typically potassium, acting as a catalysis promoter. In the practice of the method of said invention, a catalyst life extender is supplied to at least one reaction chamber used to prepare the vinyl aromatic hydrocarbon. The catalyst life extender is a compound containing potassium and is neither excessively deliquescent nor dangerously reactive, and has a melting point or vapor point such that it can be used at normal process temperatures without blocking lines or fouling process equipment. Preferably, the catalyst life extender is a potassium salt of a carboxylic acid. More preferably, the catalyst life extender is selected from the group including the potassium salts of mono-carboxylic acids having from 2 to about 10 carbons. Most preferably, the catalyst life extender is potassium acetate. The potassium carboxylates useful with said invention are preferably vaporous under process conditions within the processes wherein they are used. The potassium carboxylates are injected using steam as a source of heat and vaporization. It is important that the potassium carboxylates be sufficiently stable that the potassium carboxylates, or at least their stable potassium bearing decomposition products, function to deliver potassium to the dehydration catalysts. In an alternative embodiment, the catalyst life extenders of said invention are introduced into a vinyl aromatic hydrocarbon process by continuously injecting the catalyst life extender directly into the process feed stream.

It has now been discovered that the dehydrogenation of an alkylaromatic hydrocarbon to produce a vinylaromatic hydrocarbon on a catalyst consisting essentially of iron oxide promoted with an alkali metal can be improved by addition of an effective amount of an alkali metal compound in the form of a powder on said catalyst. Said powder is advantageously injected in at least one of the feedstocks sent to the dehydrogenation catalyst. Said addition is made either continuously or intermittently. The prior art has only exemplified injection in an aqueous form or as a vapor and teaches addition in aqueous, solid, vapor or in powder form as equivalents. Inventors have made many experiments and discovered that injection in the liquid form leads to an irregular addition of alkali metal. The injection in the vapor form is very difficult to carry-out.

### [Brief description of the invention]

The present invention relates to a process for catalytically converting an alkylaromatic hydrocarbon into a vinylaromatic hydrocarbon by directing said alkylaromatic hydrocarbon and steam into a reactor containing dehydrogenation catalyst, said process comprising the steps of:
(a) forming a mixed reactant stream consisting essentially of said alkylaromatic hydrocarbon and steam;
(b) bringing said mixed reactant stream into contact with a dehydrogenation catalyst consisting essentially of iron oxide catalyst promoted with alkali metal and at conditions effective to convert at least a portion of the alkylaromatic hydrocarbon to vinylaromatic hydrocarbon;
wherein an effective amount of an alkali metal compound in the form of a powder is injected continuously or intermittently in at least one of the feedstocks sent to the dehydrogenation catalyst; said effective amount of alkali metal compound being sufficient to maintain substantially constant levels of conversion of alkylaromatic hydrocarbon and selectivity of vinylaromatic hydrocarbon.

### [Detailed description of the invention]

The process of this invention broadly comprises regenerating and/or stabilizing the activity of a dehydrogenation catalyst used in the catalytic dehydrogenation of an alkylaromatic hydrocarbon to obtain a specific desired alkenylaromatic hydrocarbon.

**As regards the dehydrogenation process**, the dehydrogenation catalysts are well known in the art and are commercially available. The present invention is particularly useful with processes wherein the catalyst is one containing iron and at least one alkali metal compound, typically potassium, acting as a catalysis promoter. Such dehydrogenation catalysts are well known in the art and some of those that are available commercially include: the S6-20, S6-21 and S6-30 series from BASF Corporation; the C-105, C-015, C-025, C-035, and the FLEXICAT series from CRI Catalyst Company, L.P.; and the G-64, G-84 and STYROMAX series from Sud Chemie, Inc. All of such and similar catalysts are considered within the scope of this invention.

The catalysts listed above can typically contain, by weight, from about 40 to about 80 percent Fe₂O₃, from about 5 percent to about 30 percent K₂O, and other catalysis promoters. While these are typical catalysts used in processes that can benefit from the method of the present invention, the method of the present invention can be used with any process to prepare vinyl aromatic hydrocarbons wherein the life of the catalyst or catalysts for that process can be extended by supplying an alkali metal compound in the form of a powder in at least one of the feedstocks sent to the dehydrogenation catalyst.

In general, such catalytic dehydrogenation processes are carried out at temperatures ranging from about 400°C to about 700°C. and preferably between about 500°C to 700°C, by contacting a preheated feedstream, containing a mixture of the alkylaromatic hydrocarbon and steam, with a particular dehydrogenation catalyst. The process can be carried out in single or multistage reactors having fixed catalyst beds or in fluidized beds. The choice of starting alkylaromatic hydrocarbon, dehydrogenation catalyst, reaction temperature and proportion of alkylaromatic hydrocarbon to steam in the feedstream will affect, in part, the resulting alkenylaromatic hydrocarbon as well as the efficiency and selectivity of the conversion process.

In particular, using the aforementioned process, ethylbenzene is converted to styrene by contact with a dehydrogenation catalyst containing iron and at least one alkali metal compound. For example, the ethylbenzene-to-styrene conversion can be advantageously carried out at reaction temperatures ranging from about 500°C to about 700°C, preferably from about 550°C. to about 650°C, and at reaction pressures ranging from about 0.1 bar to about 2 bar, preferably from about 0.3 bar to about 0.5 bar (unless otherwise specified these pressures are absolute pressures). The steam-to-hydrocarbon ratio in the reactant feedstream may range from a ratio of about 0.6:1 to about 3:1 steam to ethylbenzene by weight, preferably a ratio of about 1.0:1 to about 2.0:1 steam to ethylbenzene by weight. The space velocity may range from about 0.2 to about 1.2 kilograms of ethylbenzene per hour per kilogram of catalyst.

Beginning the ethylbenzene-to-styrene conversion process with fresh catalyst, following start-up there is typically an initial conditioning period lasting from about 3-45 days characterized by high initial activity followed by rapid deactivation. For example, during the initial conditioning period, the overall level of conversion of ethylbenzene to styrene might drop off to below about 55 mole % and the level of styrene selectivity might fall to below about 93 mole %. Thereafter in conventional ethylbenzene-to-styrene dehydrogenation processes, the level of catalyst activity continues to fall albeit at slower rates than during the initial conditioning period. In a multi-stage reactor, the level of conversion of ethylbenzene to styrene in each stage might drop by about one-third, for example, from about 30-36 mole % to about 20-24 mole %, during the initial conditioning period, and then continue to drop at a slower rate thereafter. The end of the initial conditioning period in this process generally can be identified by those skilled in the art as the point at which the slope of the line plotting conversion level over time flattens out. As noted previously, the prior art has suggested a number of possible explanations for the gradual deterioration of catalyst activity, but no single mechanism seems to fully account for this phenomenon.

Whatever the explanation, the continued process deterioration beyond the initial conditioning period leads to a number of problems and disadvantages. First, the efficiency of the conversion process is reduced. Unreacted ethylbenzene must be separated from the other components of the output stream for recycling. Styrene must similarly be separated from the unreacted ethylbenzene as well as from other reaction products. Second, instead of a relatively uniform output stream having relatively constant ratios of styrene, ethylbenzene and miscellaneous by-products, process deterioration leads to an output stream of ever-changing composition. Third, at some point, the level of conversion or the level of styrene selectivity or both fall sufficiently low that the process is no longer economically viable. At this point, the process would have to be shut down and the catalyst either replaced or regenerated by conventional means.

The method in the process of the present invention is capable of restoring and/or stabilizing the activity of the catalyst and consequently extending the life of the catalyst. More particularly, the method of this invention is capable of restoring the activity of the dehydrogenation catalyst to substantially the same high levels of conversion and selectivity as those established at the end of the initial conditioning period. The method of this invention is also capable of stabilizing the activity of the catalyst at those same high levels of conversion and selectivity for extended periods of time beyond those achievable with the conventional increase of the dehydrogenation temperature. Said increase of the dehydrogenation temperature can be made, for example, by increasing the temperature of steam or by adding heat to the dehydrogenation reactor. The method of this invention may also be utilized in tandem with the aforedescribed temperature-raising technique for additional benefits of further increasing the catalyst life or increasing the ethylbenzene conversion.

**As regards the injection of the alkali metal compound,** the method in the process of the present invention is characterized in that an effective amount of an alkali metal compound in the form of a powder is injected continuously or intermittently in at least one of the feedstocks sent to the dehydrogenation catalyst; said effective amount of alkali metal compound being sufficient to maintain substantially constant levels of conversion of alkylaromatic hydrocarbon and selectivity of vinylaromatic hydrocarbon.

The term "maintain" as used herein is intended to be construed to mean "to keep in a state of repair, efficiency or validity; to preserve from failure or decline over a protracted period of time, e.g. for many months or years." The method is of particular utility in connection with regenerating and/or stabilizing a dehydrogenation catalyst containing iron and at least one alkali metal compound.

Advantageously the feedstocks, steam and alkylaromatic hydrocarbon, are mixed before contacting the dehydrogenation catalyst. The powder can be injected in said mixed stream. The powder can also be injected in a stream passing between the stages of a multi-stage dehydrogenation reactor. Addition of alkali metal compound in the form of a powder to the reactant stream in accordance with the process of this invention may be advantageously employed between some or all of the reactor stages in a multi-stage reactor.

The powder can be injected by any means. By way of example the powder is in a vessel under a nitrogen pressure, said vessel is connected to the pipe in which the powder has to be injected. At least there is a valve on the pipe between the pressurized vessel and the pipe where the powder is injected, said valve is open during injection. Of course the nitrogen pressure is higher than the pressure in the pipe where the powder is to be injected. Said apparatus is known per se. Amount of injected powder is controlled by any means such as a metering valve, a restricted orifice, opening of the valve during a certain time or any combination thereof.

The process of the present invention for catalytically converting an alkylaromatic hydrocarbon into a vinylaromatic hydrocarbon may advantageously include monitoring means for monitoring the chemical composition of the streams entering or leaving the dehydrogenation reactor as well as temperatures and pressures of various streams connected to said reactor. The monitoring means may also advantageously be coupled to activating means for activating alkali metal compound supply means. The monitoring means can be adapted through conventional technology to send a signal to the alkali metal compound supply means whenever the exit stream from the subject reactor stage falls below a predetermined level of conversion or selectivity, which indicates degradation of catalyst activity in the reactor stage. Upon activation by the signal from the associated monitoring means, the alkali metal compound supply means would begin to supply alkali metal compound at a predetermined rate to the associated feedstream or reactant stream. The apparatus can be designed on activation to continue supplying alkali metal compound for a predetermined period of time or until another signal from the associated monitoring means signals that catalyst activity in the subject reactor stage has been restored to the desired activity level.

As an alternative to the automated, intermittent addition system described above, it is also within the scope of this invention to continuously add predetermined amounts of alkali metal compound to the respective feedstreams or reactant streams, or, alternatively, to add alkali metal compound in predetermined mounts and at predetermined intervals. This may be combined with continuous or intermittent monitoring of one or more reactor stage exit streams. Upon signs of catalyst degradation in any reactor stage, means for increasing the addition of alkali metal compound to a feedstream or reactant stream upstream from the subject reactor stage can be manually activated. The increased rate of addition of alkali metal compound can be for a limited period of time until the desired catalyst activity level is restored or else it can be maintained at the new, higher rate.

**As regards the alkali metal itself,** the alkali metal compounds that are useful in carrying out the method of this invention include all non-halogen sources of alkali metal ions. As used in connection with this invention, the term "alkali metal" is meant to include, but without limitation thereto, potassium, sodium, lithium and other less-common members of the group IA metals of the periodic table, such as rubidium and cesium. Cost considerations will ordinarily dictate the choice of a potassium or sodium compound, however. For some applications, members of the group IIA metals of the periodic table (e.g. magnesium, calcium, and so forth) may also have utility. Selection of an appropriate alkali metal compound is considered a matter of routine experimentation. In connection with the dehydrogenation of ethylbenzene to styrene, the preferred alkali metal compounds are potassium compounds, more particularly one or more compounds selected from the group consisting of potassium oxide, potassium hydroxide, and potassium carbonate. It is also within the scope of this invention to use mixtures of two or more alkali metal compounds. Because halogen ions, such as chloride, have typically been found to poison the dehydrogenation catalyst, alkali metal compounds such as potassium chloride should generally be avoided.

The amount of alkali metal compound to be added to a feedstream or reactant stream in accordance with this invention may vary depending upon the catalyst, the alkylaromatic hydrocarbon, the reaction conditions, and the alkali metal compound itself. An effective amount or an effective rate of addition of alkali metal compound to the reactant stream sufficient to maintain high levels of conversion and selectivity can be determined by routine experimentation in order to optimize system performance. In general, it has been found that an effective amount of the alkali metal compound comprises from about 0.01 to about 100 parts by weight of alkali metal compound per million parts of the reactant stream, preferably from about 0.10 to about 10 parts by weight, on average over a representative time frame. A representative time frame as used herein means that period of time during which high levels of conversion and selectivity are maintained without further addition of alkali metal compound. It is also within the scope of this invention to vary the amount of alkali metal compound in the reactant stream over time to restore or to maintain optimum system performance.

The alkali metal compound may be added to the reactant stream either continuously or intermittently and, if intermittently, at regular or irregular intervals. Whether the alkali metal compound is added continuously or intermittently, the amounts added and, in the case of intermittent additions, the intervals selected, should be such to insure addition of an effective amount sufficient to restore or to maintain the desired high levels of conversion and selectivity. In general, such amounts will comprise from about 0.01 to about 100 parts weight of alkali metal compound per million parts of the reactant stream on average over a representative time frame.

Advantageously the powder of alkali metal compound has a particle size ranging from 30 to 50 µm preferably from 10 to 15 µm. Advantageously 100% of the particles are under 70 µm.

## Claims

1. Process for catalytically converting an alkylaromatic hydrocarbon into a vinylaromatic hydrocarbon by directing said alkylaromatic hydrocarbon and steam into a reactor containing dehydrogenation catalyst, said process comprising the steps of:
(a) forming a mixed reactant stream consisting essentially of said alkylaromatic hydrocarbon and steam;
(b) bringing said mixed reactant stream into contact with a dehydrogenation catalyst consisting essentially of iron oxide catalyst promoted with alkali metal and at conditions effective to convert at least a portion of the alkylaromatic hydrocarbon to vinylaromatic hydrocarbon;
wherein an effective amount of an alkali metal compound in the form of a powder is injected continuously or intermittently in at least one of the feedstocks sent to the dehydrogenation catalyst; said effective amount of alkali metal compound being sufficient to maintain substantially constant levels of conversion of alkylaromatic hydrocarbon and selectivity of vinylaromatic hydrocarbon.

2. Process according to claim 1 wherein the alkylaromatic hydrocarbon is ethylbenzene and the vinylaromatic hydrocarbon is styrene.

3. Process according to claim 1 or 2 wherein the alkali metal compound is potassium carbonate.
